(19) Europäisches Patentamt
European Patent Office
Office européen des brevets

(11) EP 1 464 644 A1

(12) **EUROPEAN PATENT APPLICATION**

(43) Date of publication:
**06.10.2004 Bulletin 2004/41**

(51) Int Cl.[7]: **C07D 457/12**

(21) Application number: **04251915.7**

(22) Date of filing: **31.03.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **31.03.2003 GB 0307377**

(71) Applicant: **MeRaD Pharmaceuticals Limited
Cambridge CB3 0LH (GB)**

(72) Inventors:
- **Kumar, Raj, Dr.
Cambridge CB3 0LH (GB)**
- **Kumar, Ajay
Cambridge CB3 0LH (GB)**

(74) Representative: **Wilson Gunn M'Caw
5th Floor,
Blackfriars House,
The Parsonage
Manchester M3 2JA (GB)**

(54) **Ergoline derivatives**

(57) An ergoline derivative of formula 1:

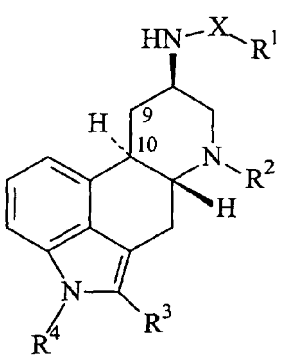

**Formula I**

wherein:

| | |
|---|---|
| X is | C=NH, C=O, C=S, $PO_2$ or $SO_2$; |
| $R^1$ is | $C_{1-7}$ alkyl, $C_{3-7}$ cycloalkyl, optionally substituted or heterocyclic fused phenyl, alkylamino or dialkylamino, $R_2$ NC(S)NH, alkoxy and alkylthio; |
| $R^2$ is | H, $C_{1-5}$ alkyl or $C_{3-5}$ alkenyl; |
| $R^3$ is | H, F, Cl, Br, I, CN, Me, $CHF_2$ or $CF_3$; |
| $R^4$ is | H or $C_{1-4}$ alkyl; |

9, 10 position are dihydro- (as shown in formula 1), didehydro-, dihydroxy-, hydroxyhalo- (unsaturated), epoxy-, dihalo-, hydrohalo-, hydrohydroxy- or CAB (A, B is halogen or hydrogen) adducts; and acid addition and metal salts thereof.

EP 1 464 644 A1

## Description

The present invention relates to ergoline derivatives.

Ergoline is a naturally occurring compound which exists as both the 8α and 8β epimers. The 8α-ergolines have been the subject of extensive research. In particular, the 8α-N-substituted ergolines comprise a major class of compounds possessing varying degrees and types of biological activity and potential therapeutic utility. However, the corresponding 8β-epimers have undergone limited study.

Novel 8β-N-substituted ergoline derivatives have now been found to possess useful and/or advantageous biological activity.

Thus, according to the first aspect of the present invention, there is provided an ergoline derivative of formula 1:

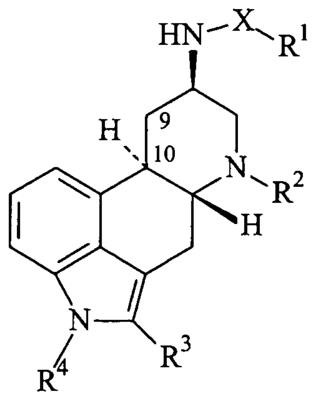

**Formula I**

wherein:

X is C=NH, C=O, C=S, $PO_2$ or $SO_2$;

$R^1$ is $C_{1-7}$ alkyl, $C_{3-7}$ cycloalkyl, optionally substituted or heterocyclic fused phenyl, alkylamino or dialkylamino, $R_2$ NC(S)NH, alkoxy and alkylthio;

$R^2$ is H, $C_{1-5}$ alkyl or $C_{3-5}$ alkenyl;

$R^3$ is H, F, Cl, Br, I, CN, Me, $CHF_2$ or $CF_3$;

$R^4$ is H or $C_{1-4}$ alkyl; 9, 10 positions are dihydro- (as shown in formula 1), didehydro-, dihydroxy-, hydroxyhalo-, epoxy-, dihalo-, hydrohalo-, hydrohydroxy- or CAB (A, B is halogen or hydrogen) adducts; and acid addition and metal salts thereof.

The compounds of the present invention have been found to be particularly useful in treating disorders of the central nervous system. For example, the compounds interact with central dopamine receptors to produce an agonistic and/or antagonistic response. Their dopamine activity (agonistic behaviour) shows them to be valuable compounds for the treatment of dopamine deficiency conditions of the central nervous system. For example, the treatment of Parkinson's disease, certain forms of dementia, hyper prolactinaemia. Their anti-dopamine activity (antagonistic behaviour) shows them to be valuable neuroleptics for the treatment of psychoses of the schizophrenic range of disorders. These conditions are given by way of example only and do not preclude other psychotic disorders and symptoms and conditions arising therefrom.

Furthermore, the compounds of the present invention have also been found to act upon central noradrenaline and serotonine receptors. This activity shows the compounds to be valuable for the treatment of memory and learning difficulties and other conditions of insufficiency due to aging.

Compounds of the present invention may also assume the role of partial agonists and antagonists at the receptor sites of biogenic amines. In keeping with their wide spectrum of pharmacological activities, the compounds of the present invention find application in the treatment of a variety of clinical conditions such as post-partum haemorrhage, migraine and other vascular headaches, orthostatic hypotension, senile cerebral insufficiency, Alzheimer's

disease and conditions associated with hyperprolactinemia and the symptoms thereof.

Compounds of the present invention can show high selectivity towards different receptor sites and in such situations be developed as drugs displaying reduced side-effects.

Additional applications of the compounds of the present invention will be apparent to those skilled in the art. Table 1 represents preferred substituents, which may be used in any combination, for use with the ergoline derivative of formula 1:-

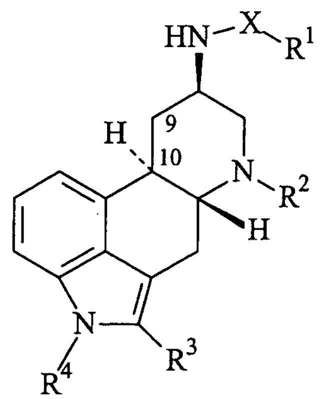

**Formula I**

Table 1

| Substituent | Chemical Moeity |
|---|---|
| X | C=O or $SO_2$ |
| $R^1$ | $C_{3-7}$ alkyl; $C_{3-7}$ cycloalkyl; phenyl; mono- or di-substituted phenyl wherein the substituent is selected from halogen, Me, Et, MeO-, EtO-, a divalent bridging residue such as epoxy or $-O-CH_2-O-$; mono- or di- $C_{1-6}$ alkyl amino; or $C_{1-6}$ alkoxy. |
| $R^2$ | $C_{2-5}$ alkyl or $C_{3-5}$ alkenyl |
| $R^3$ | H, F, Cl, Br, I, CN, Me, $CHF_2$ or $CF_3$ |
| $R^4$ | H or $C_{1-4}$ alkyl |
| 9, 10 position | di-dehydro,-dihydroxy-, hydroxyhalo-, epoxy-, dihalo-, hydrohalo-, hydrohydroxy-, or CAB (A,B is H or halogen). |

Table 2 represents the most preferred substituents, which may be used in any combination, for use with the ergoline derivative of formula 1:-

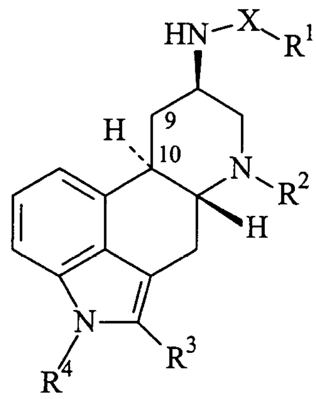

**Formula I**

Table 2

| Substituent | Chemical Moeity |
|---|---|
| X | C=O or $SO_2$ |
| $R^1$ | $C_{3-7}$ alkyl; $C_{3-7}$ cycloalkyl; phenyl; mono- or di- substituted phenyl wherein the substituent is selected from halogen, Me, Et, MeO-, EtO-, a divalent bridging residue such as epoxy or $-O-CH_2-O-$; mono- or di= $C_{1-6}$ alkyl amino; or $C_{1-6}$ alkoxy. |
| $R^2$ | Me |
| $R^3$ | F, Cl or Br |
| $R^4$ | H or $C_{1-4}$ alkyl |
| 9, 10 | Dihydro or dehydro |

The alkyl groups as referred to hereinbefore may be straight chain, cyclic or branched structures.

The compounds of the present invention may be in the form of an acid addition salt, such as for example sulphate, hydrochloride, acetate, maleate, tartrate, citrate; and they may be in the form of an alkali metal or alkaline earth salt, such as for example sodium, potassium, lithium, calcium. The compounds may also be in the form of a hydrate or solvate.

The present invention includes the compounds of the present invention in any crystalline form.

The compounds of the present invention may conveniently be prepared by reacting an appropriately substituted ergoline of formula II with an appropriately substituted compound of formula III such that a compound of the present invention is afforded.

Thus according to the second aspect of the present invention there is provided a method for the preparation of an ergoline derivative of claim 1, comprising the steps of: reacting a substituted ergoline derivative of formula I with a compound of formula III such that n ergoline derivative as hereinbefore defined is afforded.

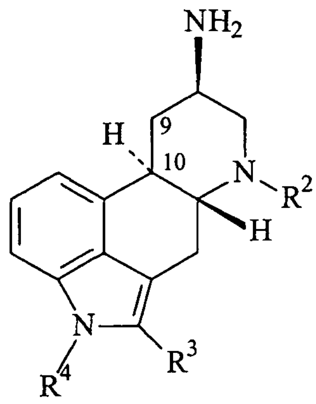

**Formula II**

wherein $R^2$, $R^3$, $R^4$ and the 9, 10 position can be any of the substituents as referred to hereinbefore.

$R^1XOH$

**Formula III**

wherein $R^1$ and X can be any of the substituents as referred to hereinbefore, or a reactive derivative thereof;

Depending upon the nature of the substitution in the desired compound, it may be necessary to manipulate the substituents following the reaction of a compound of formula II with a compound of formula III. For example, halogenation of a compound of formula I wherein $R^3$ = H such that $R^3$ = halogen; or N-alkylation of a compound of formula I wherein $R^4$ = H such that $R^4$ = alkyl.

The compound may be recovered as the free base or as the acid addition salt. This is largely dependent upon the nature and behaviour of the substituents of the compound of formula I. In some circumstances it may be easier and quicker to recover the compound as the acid addition salt even though this may involve the introduction of an additional process step.

One skilled in the art would be familiar with the standard procedures necessary to produce a compound of formula I.

According to the third aspect of the present invention there is provided a pharmaceutical composition comprising an effective amount of a compound of formula I.

The pharmaceutical composition may be administered orally, parentally (including subcutaneously, intramuscularly and intravenously) or topically. Therefore, the pharmaceutical composition may be in the form of a tablet, an oil, a cream/lotion or any other suitable form.

The effective amount of the compound of formula I may vary depending upon the route of administration, the pharmaceutical formulation, the body weight of the patient, the age and general condition of the patient and the chemical form of the compound. The dose administered is ultimately at the discretion of the medical practitioner.

The total doses may be given as a single dose, multiple doses or by intravenous infusion for any selected duration.

The pharmaceutical composition of the present invention may be presented in any suitable form, for example, a tablet, a capsule, a liquid (e.g. syrup) or intravenous injection. These pharmaceutical compositions are formulated in accordance with methods well known to those skilled in the art.

In addition to the effective amount of the compound of formula I the pharmaceutical composition may also comprise additional ingredients such as carriers, diluent, excipients, surfactants, dispersing agents, binders, flavourings or sweeteners. The choice of these ingredients is made out of consideration for the final form of the pharmaceutical composition and is limited only by their compatibility with the compound of formula I.

For example, where the composition is to take the form of an ointment the additional ingredients may include a diluent, a buffer, flavouring agent, binder, surfactant, thickener, lubricant and/or a preservative or other pharmaceutically inert excipient.

According to the fourth aspect of the present invention there is provided the use of an effective amount of a compound of formula I in the preparation of a pharmaceutical composition for the treatment of psychotic disorders.

According to a fifth aspect of the present invention there is provided the use of an effective amount of a compound of formula I in the preparation of a pharmaceutical composition for the treatment of dopamine deficiency conditions of the central nervous system.

It is envisaged the ergoline derivatives described herein may be used in combination with other therapies for the treatment of diseases such as depression, diseases of the central nervous system, Alzheimer's disease, memory loss and anxiety. These examples do not represent an exhaustive list.

It is of course to be understood that the invention is not intended to be restricted to the details of the above embodiments which are described by way of example only.

## Claims

1. An ergoline derivative of formula 1:

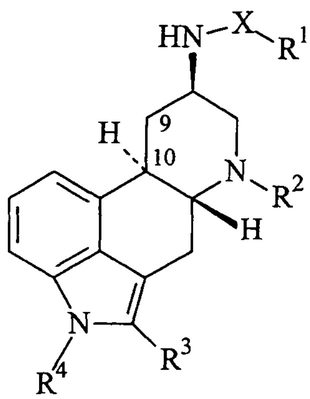

**Formula I**

wherein:

X is C=NH, C=O, C=S, $PO_2$ or $SO_2$;
$R^1$ is $C_{1-7}$ alkyl, $C_{3-7}$ cycloalkyl, optionally substituted or heterocyclic fused phenyl, alkylamino or dialkylamino, $R_2$ NC(S)NH, alkoxy and alkylthio;
$R^2$ is H, $C_{1-5}$ alkyl or $C_{3-5}$ alkenyl;
$R^3$ is H, F, Cl, Br, I, CN, Me, $CHF_2$ or $CF_3$;
$R^4$ is H or $C_{1-4}$ alkyl;

9, 10 position are dihydro- (as shown in formula 1), didehydro-, dihydroxy-, hydroxyhalo- (unsaturated), epoxy-, dihalo-, hydrohalo-, hydrohydroxy- or CAB (A, B is halogen or hydrogen) adducts; and acid addition and metal salts thereof.

2. An ergoline derivative according to claim 1, wherein:

X is C=O or $SO_2$
$R^1$ is $C_{3-7}$ alkyl; $C_{3-7}$ cycloalkyl; phenyl; mono- or di- substituted phenyl wherein the substituent is selected from halogen, Me, Et, MeO-, EtO-, a divalent bridging residue such as epoxy or -O-$CH_2$-O-; mono- or di= $C_{1-6}$ alkyl amino; or $C_{1-6}$ alkoxy.

$R^2$ is $C_{2-5}$ alkyl or $C_{3-5}$ alkenyl;
$R^3$ is H, F, Cl, Br, I, CN, Me, $CHF_2$ or $CF_3$
$R^4$ is H or $C_{1-4}$ alkyl

9, 10 position is didehydro, dihydroxy, hydroxyhalo, epoxy, dihalo-, hydrohalo-, hydrohydroxy-, or CAB (A,B is H or halogen).

**3.** An ergoline derivative according to claim 2, wherein

X is C=O or $SO_2$.
$R^1$ is $C_{3-7}$ alkyl; $C_{3-7}$ cycloalkyl; phenyl; mono- or di- substituted phenyl wherein the substituent is selected from halogen, Me, Et, MeO-, EtO-, a divalent bridging residue such as epoxy or $-O-CH_2-O-$; mono- or di= $C_{1-6}$ alkyl amino; or $C_{1-6}$ alkoxy.
$R^2$ is Me
$R^3$ is F, Cl or Br
$R^4$ is H or $C_{1-4}$ alkyl

9, 10 position is dihydro- or dehydro-.

**4.** An ergoline derivative according to any preceding claim, wherein the alkyl groups are straight chain, cyclic or branched structures.

**5.** An ergoline derivative according to any preceding claim, wherein the derivative is crystalline.

**6.** A method for the preparation of an ergoline derivative of claim 1, comprising the steps of: reacting a substituted ergoline of formula II with a compound of formula III such that an ergoline derivative as hereinbefore defined is afforded.

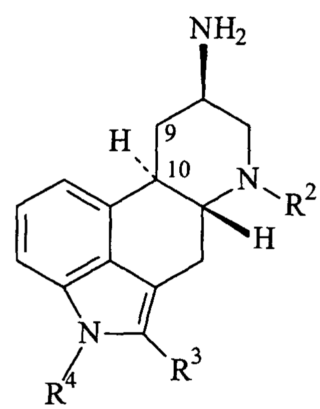

**Formula II**

wherein
$R^2$, $R^3$, $R^4$ and the 9, 10 position is any of the substituents as hereinbefore defined:
and

$R^1XOH$ formula III

wherein
$R^1$ and X is any of the substituents as hereinbefore defined.

**7.** A pharmaceutical composition comprising an effective amount of a compound of formula I.

**8.** A composition according to claim 7, wherein the composition further comprises additional ingredients, either alone or in combination, selected from carriers, diluents, excipients, surfactants, dispersing agents, binders, flavourings and sweeteners.

**9.** The use of an effective amount of a compound of formula I in the preparation of a pharmaceutical composition for the treatment of psychotic disorders.

**10.** The use of an effective amount of a compound of formula I in the preparation of a pharmaceutical composition for the treatment of dopamine deficiency conditions of the central nervous system.

European Patent Office

# EUROPEAN SEARCH REPORT

Application Number
EP 04 25 1915

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 021 206 A (SCHERING AG) 7 January 1981 (1981-01-07) * page 2, line 27 - line 29; claims 2,4,6,8,10,11,13,15,17,20,22,24,26,28,30,32,34,36; examples 1-5,7,9,11,13 * ----- | 1-10 | C07D457/12 |
| X | EP 0 056 358 A (SCHERING AG) 21 July 1982 (1982-07-21) * claims 1,6,8-11; examples 3,6,-9,12 * ----- | 1-10 | |
| X | PORTER R H P ET AL: "Functional characterization of agonists at recombinant human 5-HT2A, 5-HT2B and 5-HT2C receptors in CHO-K1 cells" BRITISH JOURNAL OF PHARMACOLOGY, BASINGSTOKE, HANTS, GB, vol. 128, 1999, pages 13-20, XP002962670 ISSN: 0007-1188 * table 1, (S)-lisuride * ----- | 1,7,9 | |
| X | EP 0 082 808 A (SCHERING AG) 29 June 1983 (1983-06-29) * claims 1,25,26; examples 6,8,10,12 * ----- | 1-10 | TECHNICAL FIELDS SEARCHED (Int.Cl.7) C07D |
| X | BERNARDI, L. ET AL: "Ergolines. III. D-Dihydrolysergic acid II derivs" GAZZETTA CHIMICA ITALIANA , 94(8-9), 955-60 CODEN: GCITA9; ISSN: 0016-5603, 1964, XP008031769 * table I (1-3,5,7) ** table I * ----- | 1 | |

The present search report has been drawn up for all claims

| Place of search | Date of completion of the search | Examiner |
|---|---|---|
| The Hague | 17 June 2004 | Alfaro Faus, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 25 1915

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2004

| Patent document cited in search report | | Publication date | Patent family member(s) | | | Publication date |
|---|---|---|---|---|---|---|
| EP 0021206 | A | 07-01-1981 | DE | 2924102 | A1 | 18-12-1980 |
| | | | DE | 3016691 | A1 | 29-10-1981 |
| | | | AT | 3864 | T | 15-07-1983 |
| | | | AU | 541679 | B2 | 17-01-1985 |
| | | | AU | 5913580 | A | 18-12-1980 |
| | | | CA | 1140922 | A1 | 08-02-1983 |
| | | | CS | 223809 | B2 | 25-11-1983 |
| | | | DD | 151449 | A5 | 21-10-1981 |
| | | | DE | 3063869 | D1 | 28-07-1983 |
| | | | DK | 252680 | A ,B, | 14-12-1980 |
| | | | EP | 0021206 | A1 | 07-01-1981 |
| | | | ES | 8101073 | A1 | 01-03-1981 |
| | | | HU | 184786 | B | 29-10-1984 |
| | | | IE | 49991 | B1 | 22-01-1986 |
| | | | IL | 60261 | A | 31-10-1983 |
| | | | JP | 1456316 | C | 09-09-1988 |
| | | | JP | 56002982 | A | 13-01-1981 |
| | | | JP | 63002265 | B | 18-01-1988 |
| | | | SU | 965356 | A3 | 07-10-1982 |
| | | | US | 4379790 | A | 12-04-1983 |
| | | | YU | 154480 | A1 | 30-09-1983 |
| EP 0056358 | A | 21-07-1982 | DE | 3101535 | A1 | 12-08-1982 |
| | | | AT | 26716 | T | 15-05-1987 |
| | | | AU | 547987 | B2 | 14-11-1985 |
| | | | AU | 7929882 | A | 22-07-1982 |
| | | | CA | 1175043 | A1 | 25-09-1984 |
| | | | CS | 227030 | B2 | 16-04-1984 |
| | | | CS | 227048 | B2 | 16-04-1984 |
| | | | DD | 202027 | A5 | 24-08-1983 |
| | | | DE | 3276114 | D1 | 27-05-1987 |
| | | | DK | 13382 | A ,B, | 15-07-1982 |
| | | | EP | 0056358 | A1 | 21-07-1982 |
| | | | ES | 8304127 | A1 | 16-05-1983 |
| | | | ES | 8307802 | A1 | 01-11-1983 |
| | | | GR | 75159 | A1 | 13-07-1984 |
| | | | HU | 186898 | B | 28-10-1985 |
| | | | IE | 52849 | B1 | 30-03-1988 |
| | | | JP | 1752276 | C | 08-04-1993 |
| | | | JP | 4037079 | B | 18-06-1992 |
| | | | JP | 57145875 | A | 09-09-1982 |
| | | | SU | 1097199 | A3 | 07-06-1984 |
| | | | US | 4853390 | A | 01-08-1989 |
| | | | YU | 307181 | A1 | 31-12-1983 |
| EP 0082808 | A | 29-06-1983 | DE | 3151912 | A1 | 30-06-1983 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 25 1915

This annex lists the patent family members relating to the patent documents cited in the above-mentioned European search report. The members are as contained in the European Patent Office EDP file on
The European Patent Office is in no way liable for these particulars which are merely given for the purpose of information.

17-06-2004

| Patent document cited in search report | Publication date | Patent family member(s) | Publication date |
|---|---|---|---|
| EP 0082808 A | | AT 27458 T | 15-06-1987 |
| | | AU 570621 B2 | 24-03-1988 |
| | | AU 9146882 A | 30-06-1983 |
| | | CA 1319684 C | 29-06-1993 |
| | | CS 8209553 A2 | 13-02-1984 |
| | | DD 208619 A5 | 04-04-1984 |
| | | DE 3276433 D1 | 02-07-1987 |
| | | DK 566982 A ,B, | 24-06-1983 |
| | | EP 0082808 A1 | 29-06-1983 |
| | | EP 0185392 A2 | 25-06-1986 |
| | | ES 8400115 A1 | 01-01-1984 |
| | | GR 78410 A1 | 27-09-1984 |
| | | HU 191623 B | 30-03-1987 |
| | | IE 56541 B1 | 28-08-1991 |
| | | IL 67545 A | 30-09-1986 |
| | | JP 1684344 C | 31-07-1992 |
| | | JP 3052463 B | 12-08-1991 |
| | | JP 58150593 A | 07-09-1983 |
| | | RO 85270 A1 | 29-09-1984 |
| | | RO 87945 A1 | 30-09-1986 |
| | | SU 1313348 A3 | 23-05-1987 |
| | | US 4826852 A | 02-05-1989 |
| | | YU 282382 A1 | 31-12-1986 |

EPO FORM P0459

For more details about this annex : see Official Journal of the European Patent Office, No. 12/82